Europäisches Patentamt

European Patent Office

Office européen des brevets

(11) Publication number: 0 369 553
A1

# (12) EUROPEAN PATENT APPLICATION

(21) Application number: 89202907.5

(51) Int. Cl.5: **C12P 41/00, C12P 7/40**

(22) Date of filing: 17.11.89

(30) Priority: 18.11.88 NL 8802849

(43) Date of publication of application:
23.05.90 Bulletin 90/21

(84) Designated Contracting States:
AT BE CH DE ES FR GB GR IT LI NL SE

(71) Applicant: STAMICARBON B.V.
Mijnweg 1
NL-6167 AC Geleen(NL)

(72) Inventor: Elferink, Vincentius Henricus Maria
Esstraat 33
NL-7131 CT Lichtenvoorde(NL)
Inventor: Kierkels, Johannes Gerardus
Theodorus
Bernhardlaan 38
NL-6133 BK Sittard(NL)
Inventor: Kloosterman, Marcel
Lienaertsstraat 180
NL-6164 GL Geleen(NL)
Inventor: Roskam, Jan Hendrik
Hugo de Grootstraat 10
NL-5462 CM Veghel(NL)

(54) Process for the enantioselective preparation of D-(-)-3-hal-2-methylpropionic acid or derivatives thereof and the preparation of captopril therefrom.

(57) The invention relates to the enantioselective preparation of D-(-)-3-Hal-2-methylpropionic acid or esters thereof, Hal being a halogen atom, preferably a chlorine or bromine atom, by enantioselective enzymatic hydrolysis of an ester of the aforementioned acid with an enzyme preparation so that, at a degree of ester conversion of at most 75%, the residual ester fraction contains an enantiomeric excess of D-ester of at least 95%.

EP 0 369 553 A1

# PROCESS FOR THE ENANTIOSELECTIVE PREPARATION OF D-(-)-3-HAL-2-METHYLPROPIONIC ACID OR DERIVATIVES THEREOF AND THE PREPARATION OF CAPTOPRIL THEREFROM

The present invention relates to a process for the enan-derivatives thereof, and also to the preparation of Captopril therefrom. Hal indicates a halogen atom, preferably a chlorine or bromine atom.

Captopril is the common name for N-(3-mercapto-2-D-methylpropanoyl)-L-proline, which is also commercially available under the registered tradename 'Capoten'R. For the sake of simplicity the name Captopril will be used to indicate the aforementioned compound in this description and the appurtenant claims.

Captopril has a very strong inhibiting effect on angiotensin converting enzyme, for which reason it is referred to as an ACE-inhibitor, and as such it is active against hypertension when administered orally. The so-called ACE-inhibiting effect is greatly dependent on the spatial configuration of the molecule. The proline radical in the Captopril molecule comes from the L-proline. The 3-mercapto-2-methylpropionic acid radical in Captopril comes from the D-enantiomer. Captopril is about 100 times as active as the stereoisomeric compound in which the L-enantiomer of 3-mercapto-2-methylpropionic acid is bound. According to the R-S convention, D-3-mercapto-2-methylpropionic acid or D-(-)-3-Hal-2-methylpropionic acid is an S-enantiomer.

A synthesis of Captopril is known from a publication by Masami Shimazaki et al. in Chem. Pharm. Bull. 30 (1982) 3139-3146, in which optically active 3-hydroxy-2-methylpropionic acid is used as starting material. This publication and NL-A-80.06026 (which corresponds to US-A-4.310.635 and other patents) also state that the D-enantiomer of that acid can be prepared by subjecting isobutyric acid, methacrylic acid or a mixture of those acids to the action of a microorganism that is capable of converting the aforementioned acids into D-3-hydroxy-2-methylpropionic acid. Because the aforementioned hydroxy acid cannot be converted directly into the corresponding mercapto-compound, it is converted into D-3-chloro-2-methylpropionyl chloride with thionyl chloride. The D-3-chloro-2-methylpropionyl chloride can then be used as starting compound for the preparation of Captopril. The preparation of Captopril proceeds via the D-enantiomer of a 3-alkanoylthio- or 3-arylthio-2-methylpropionic acid or a derivative thereof, in particular an ester thereof. Such a compound is coupled with L-proline and then the thiol group is released from the coupling product by the removal of the alkanoyl or aroyl group. A disadvantage of this synthesis is that 3-hydroxy-2-methylpropionic acid is unstable and that the enzymatic conversion of isobutyric acid and/or methcrylic acid into D-3-hydroxy-2-methylpropionic acid is a difficult and expensive process, as is also mentioned in WO-A-87/05328.

EP-A-130.752; WO-A-87/05328 and a publication by Qu-Ming Gu et al. in Tetrahedron Letters 27 (1986) 5203-5206 describe the preparation of enantiomers from 3-alkanoylthio- or 3-aroylthio-2-methylpropionic acid by enzymatic hydrolysis of esters thereof. Enzymatic hydrolyses are usually not completely enantioselec tive reactions and, in order to obtain the desired enantiomer with a satisfactory enantiomeric purity, the hydrolysis must be terminated once a certain degree of conversion has been reached. This is known from e.g. the aforementioned publication by Qu-Ming Gu et al. and has been described more generally in a publication by Ching-Shih Chen et al. in J. Am. Soc. 104 (1982) 7294-7299.

Enantiomeric excess, which is a measure of the enantiomeric purity and is often referred to as 'e.e', is a commonly used quantity, which is defined in e.g. the publication by Ching-Shih Chen (loc. cit.), which publication is understood to be included here. In short, enantiomeric excess equals the difference of the amounts of enantiomers divided by the total of the amounts of enantiomers, which quotient can be expressed as a percentage by multiplication by 100.

For an enantioselective enzymatic hydrolysis to yield a product with a great enantiomeric purity, it will have to be terminated at less than 50% conversion. In order to obtain, on the other hand, a residual substrate (in this case ester) with a great enantiomeric purity, the hydrolysis will have to be continued to a degree of conversion of more than 50%.

A disadvantage of the preparation of enantiomers of 3-alkanoylthio- or 3-aroylthio-2-methylpropionic acid or esters thereof by enzymatic hydrolysis of the corresponding esters is that a by-product is obtained in which mainly undesired enantiomer of the acid is present or is bound as ester, but in which also a considerable amount of desired enantiomer is present or is bound as ester. The 3-alkanoylthio- and 3-aroylthio-2-methylpropionic acid can be recovered only with difficulty and at a considerable cost, which means that the by-product will have to be discharged as waste, which means a considerable burden on the environment. Moreover, the aforementioned compounds contained in the by-product are expensive and the loss thereof greatly increases the cost price.

It is commonly known - and also mentioned in the publication by Ching-Shih Chen (loc. cit.) - that the enzymatic hydrolysis of the enantiomers of a chiral ester can proceed at different rates. The greater the difference in hydrolysis rates, the greater the degree of separation of the enantiomers and the yield of an enzymatic enantioselective hydrolysis, in other words the enantioselectivity. The difference in hydrolysis rates of a hydrolysis employing a particular enzyme can be influenced to a certain extent by choosing suitable process parameters. Of course the aim will be to choose the most favourable process parameters when using a particular enzyme; this can be done with the aid of experiments designed for this purpose.

It has now been found that enantioselective D-(-)-3-Hal-2-methylpropionic acid or derivatives thereof can be prepared by - and this characterizes the invention - subjecting an ester of 3-Hal-2-methylpropionic acid, Hal being a halogen atom, and preferably a chlorine or bromine atom, to the action of an ester-bond-hydrolyzing enzyme preparation so that, at a degree of ester conversion of at most 75%, the remaining ester fraction has an enantiomeric excess of D-ester of at least 95%, and then recovering the ester fraction from the reaction product. The ester fraction with an enantiomeric excess of D-ester of at least 95% can then be used as starting product for the preparation of Captopril. If so desired, the ester can be hydrolyzed to the corresponding enantiomer of the acid. This hydrolysis can be effected by both enzymatic and chemical hydrolysis with an acid or a base or in any other suitable manner, provided that no racemization or elimination takes place.

It will be obvious to a person skilled in the art that the enantioselective hydrolysis of an ester of 3-Hal-2-methylpropionic acid can also be carried out with an enzyme preparation with less enantiospecificity, which, at 75% conversion, results in ester with an enantiomeric excess of less than 95%, or in which case, in order to obtain an ester with an enantiomeric excess of 95%, the degree of conversion must exceed 75%. Such a process is less attractive from an economic point of view, particularly if the ester of D-(-)-3-Hal-2-methylpropionic acid is to be used as starting product for the preparation of Captopril. No exclusive rights are therefore claimed for such a process.

An important advantage of the process according to the invention is that the starting product, a racemic mixture of an ester of 3-Hal-2-methylpropionic acid, can be prepared by hydrohalogenation of esters of methacrylic acid. It is also possible to hydrohalogenate methacrylic acid, after which the 3-Hal-2-methylpropionic acid obtained can be esterified by the usual esterification methods. The starting compounds are cheap and easily obtainable.

In general, of course, it does not matter what ester is used as starting material in the present process. In essence, any ester may be used, provided that the hydrocarbon radical bound in the ester does not interfere with the enzymatic enantioselective hydrolysis. The hydrocarbon radical bound in the ester may be substituted if so desired, in particular by activating substituents such as halogen atoms. For economic and other reasons it will in practice be preferable to use $C_1$-$C_4$ alkyl esters such as methyl or ethyl esters, which alkyl group may be substituted by an activating substituent if so desired. An activating substituent is capable of accelerating the enzymatic hydrolysis of the ester. For example, an ester of 2-chloroethanol can be hydrolyzed more easily and faster than an ester of ethanol. This aspect may be important for optimising the process according to the invention.

D-(-)-3-Hal-2-methylpropionic acid or an ester thereof can be converted in a known manner into the corresponding 3-alkanoylthio- or 3-aroylthio-compound, after which Captopril can be synthesized therefrom in a known manner. Alkanoylthio and aroylthio are in this description understood to include aralkanoylthio and alkaroylthio, respectively. To this effect, D-(-)-3-Hal-2-methylpropionic acid or an ester thereof must contain an enantiomeric excess that is at least equal to the enantiomeric excess of N-(3-mercapto-2-D-methylpropanoyl)-L-proline required in Captopril. That enantiomeric excess must be at least 95%, preferably at least 98% and more in particular at least 99%. Such an enantiomeric excess is therefore also desirable, or required, in the case of the D-(-)-3-Hal-2-methylpropionic acid or the ester thereof to be used. Another important advantage of the present invention is that from D-(-)-3-Hal-2-methylpropionic acid or an ester thereof (e.e. at least 95%) the corresponding enantiomer of a 3-alkanoylthio- or 3-aroylthio-2-methylpropionic acid (or an ester thereof) can be prepared. This eliminates the need to separate the enantiomers of those 3-alkanoylthio- or 3-aroylthio-compounds with all the associated disadvantages.

As is also apparent from the above, enzymatic hydrolysis of esters is commonly known and many enzymes belonging to the class of hydrolases may be used for that purpose, for example esterases such as pig's liver esterase, choline esterases, lipases, such as pig's pancreas lipase, proteolytic enzymes, etc. A number of hydrolyzing enzymes are produced on a technical scale and are commercially available. Hydrolyzing enzymes are not only obtainable from bacteria, but also from yeast and/or fungi species, from plants and animal organs such as pigs' livers, pigs' pancreas, etc.

An ester-hydrolyzing enzyme preparation in general, but also an ester-bond-hydrolyzing enzyme preparation as is used in the present invention, is not limited by source, purity, etc. and may consist of, for example, whole cells of microorganisms, but also of reduced cells or of extracts thereof. Both free and immobilized enzyme may be used. The invention is not limited by the form in which the enzyme is contained in the enzyme preparation.

Esters of 3-Hal-2-methylpropionic acid cannot be hydrolyzed with any randomly selected ester-hydrolyzing enzyme preparation. No or virtually no hydrolysis is effected with many commercially available enzyme preparations, nor with many microorganisms, for example with various lipases (Candida lipolytica, Amano; Penicillium roqueforti, Amano; wheat germ, Sigma), esterases (pregastric esterase, Amano; cholesterol esterase from bovine pancreas, Sigma), proteases (Fungal protease, Miles Kali) and others.

Many other preparations, on the other hand, are capable of hydrolyzing esters of 3-Hal-2-methylpropionic acid but in many cases the condition is not met that, at a degree of ester conversion of at most 75%, the enantiomeric excess of D-ester in the non-hydrolyzed ester fraction must be at least 95%. For example, investigations carried out by the applicant have shown that the enantioselectivity of many enzyme preparations that are commercially available, such as various esterases (pig's liver, Sigma; butyryl cholinesterase from human serum; Sigma), acylases (porcine kidney, Sigma; type I from Aspergillus niger, Sigma), lipases (Humicola lanuginosa, Biocatalyst; Penicillium cyclopium, Amano; lipoprotein, Amano) proteases (Maxatase, Gist-Brocades; Optimase L 660, Miles Kali) and others, is insufficient to meet the requirements of the hydrolysis of esters of 3-Hal-2-methylpropionic acid. Various other microorganisms also hydrolyse esters of 3-Hal-2-methylpropionic acid with insufficient selectivity. Some examples are: Flavobacterium aquatile, Azotobacter agi lis, Arthrobacter globiformis, Hansenula polymorpha, Geotrichum candidum, Torula monosa, Kluyveromyces fragilis, Pichia pinus, Trichosporon cutaneum, Zygosaccharomyces barkeri, Saccharomyces cerevisiae and others.

Surprisingly, it has now been found that a number of preparations do present sufficient selectivity, namely commercially available lipases of the fungi Rhizopus (e.g. delemar, niveus, javanicus, arrhizus, japonicus) and Mucor (e.g. javanicus, miehei), the lipase of the yeast Candida (e.g. cylindraceae) and the following microorganisms from strain collections: Verticillium glaucum NV DSM 2011, Gliocladium viride CBS 22848, Rhodococcus erythropolis NCIB 11538, Rhodococcus erythropolis NCIB 11539, Rhodococcus erythropolis NCIB 11540, Rhodococcus rhodochrous ATCC 21243, Rhodococcus rhodochrous ATCC 21197, Rhodococcus sp. ATCC 4275, Rhodococcus sp. ATCC 19071, Rhodococcus sp. ATCC 19148 Nocardia tartaricans ATCC 31190, Xanthomonas hyacinthi NV DSM 49 (NCIB 40073), Pseudomonas fluorescens NV DSM 91 (NCIB 40074), Bacillus cereus NV DSM 6 (NCIB 40072), Corynebacterium bovis ATCC 7715, Mycobacterium phlei ATCC 15610, Strain TA-2 (isolated on triacetin) (NCIB 40069), Strain TB-2 (isolated on tributyrin) (NCIB 40070), Strain EE-1 (NCIB 40071), Erwinia herbicola ATCC 14589.

Enzyme preparations are obtained by culturing suitable microorganisms in media containing components such as carbon nutrient, nitrogen nutrient, vitamins, minerals, etc. In order to optimise the activity and selectivity of such enzyme preparations a small amount of the ester to be hydrolyzed or a different additive often used in this type of culturing processes, such as Tween 80, etc, may be added to the culture medium.

The reaction conditions of the hydrolysis are not very critical. Usually the hydrolysis is carried out in an aqueous medium, but mixtures of water and organic solvents may also be used. If as ester-hydrolyzing enzyme a lipase is used, it is observed that a mixture of water and a hydrophobic solvent leads to excellent enantioselectivity. The pH is set at a value within the activity range of the enzyme, which is usually from 2 to 11 and preferably from 5 to 9. The hydrolysis is usually effected at ambient temperature or at a slightly elevated temperature, but reduced temperatures of -10°C to -20°C, but also moderately elevated temperatures of up to about 60°C and, in the case of thermostable enzymes, even higher temperatures may also be used. Of course the reaction conditions are chosen so that no interfering side reactions take place.

The hydrolysis can be carried out by adding the ester to the culture medium at the beginning of, during or after the culturing of the microorganism in question. Cultured cells may also be harvested, for example by centrifugation, and added to the ester-containing reaction medium. In this case use may be made of both cell suspensions and dried cells, such as lyophilized cells, spray-dried cells, or cells that have been treated with an organic solvent such as acetone or toluene. Reduced cells or cell extracts may also be used.

In general, the hydrolysis is carried out so that after the mixing of the ester and the enzyme and the addition of a buffer, the pH is kept constant within the activity range of the enzyme by means of titration with a base. The amount of base consumed is a measure of the progress of the hydrolysis. The hydrolysis is stopped when the desired degree of hydrolysis has been reached and the residual ester is recovered from the reaction mixture, for example by extraction.

The enantiomeric excess of the non-hydrolyzed ester fraction of an ester of 3-Hal-2-methylpropionic acid increases as the conversion progresses. By allowing the hydrolysis to continue for a long time, and the conversion to progress to a high degree, a substantially enantiomerically enriched ester is obtained, but only at the expense of the yield. A shorter hydrolysis with a lower degree of conversion results in a higher yield of non-hydrolyzed ester, but the ester is less enantiomerically enriched. For a person skilled in the art it will be quite easy to choose the optimum embodiment, but it is of course of great importance that an enzyme preparation is used that will cause the hydrolysis to proceed with the greatest possible enantioselectivity. It has already been mentioned that the enantioselectivity must be at least so great that, at a degree of ester conversion of at most 75%, the residual ester fraction has an enantiomeric excess of D-ester of at least 95%. Preferably, the enantiomeric excess is at least 98% and more in particular at least 99%. For the economy of the process it is desirable that the degree of conversion at which the desired enantiomeric excess is reached is as low as possible. This degree of conversion may be at most 75%.

The D-(-)-3-Hal-2-methylpropionic acid prepared according to the process according to the present invention or an ester thereof can be converted in a known manner into the corresponding D-3-acylthio-2-methylpropionic acid or an ester thereof. The type of acylthio radical to be added does not matter much and it may be of an aliphatic, an aromatic or a mixed aliphatic/aromatic nature. In general, a relatively cheap and readily available compound will be chosen. Preferably, thioacetic acid is used. The D-3-acylthio-2-methylpropionic acid or ester thereof thus obtained, preferably D-3-acetylthio-2-methylpropionic acid or an ester thereof, can be converted into Captopril in a manner known per se (see Chem. Pharm. Bull. 30 (1982), 3139-3146). This is a very attractive manner of preparing Captopril, with which the aforementioned disadvantages involved in the preparation from a racemic mixture of D-3-acylthio-2-methylpropionic acid or esters thereof are avoided.

ACE-inhibiting compounds related to Captopril are known, which differ from Captopril in that these contain a bound D-3-acylthio-2-methylpropionic acid radical instead of a D-3-mercapto-2-methylpropionic acid radical. Other compounds related to Captopril are known which, instead of proline, contain a different bound amino acid. This amino acid may be a substituted proline, a picolinic acid, whether or not substituted, or any other amino acid. A number of compounds resembling Captopril are known from e.g.: Matthew J. Wyvratt and Arthur A. Patchett, Medicinal Research Reviews 5 (1985) 483-531; and also from: Drugs of the future 11 (1986), 116-120, and 12 (1987), 485-486.

For the preparation of all such compounds with a D-3-mercapto-2-methylpropionic acid radical or a D-3-acylthio-2-methylpropionic acid radical in the molecule use may with particular advantage be made of a D-(-)-3-Hal-2-methylpropionic acid or an ester thereof prepared according to the invention. Of course, the nature of the amino acid coupled to the D-3-mercapto or acylthio-2-methylpropionic acid is essentially of no importance. If the compound related to Captopril contains a D-3-acylthio-2-methylpropionic acid radical it may be advantageous to convert the D-(-)-3-Hal-2-methylpropionic acid or an ester thereof into D-3-acylthio-2-methylpropionic acid with the same acylthio radical as in the compound to be prepared.

The invention will be further elucidated with the following examples, without being limited thereto.

The results of the examples are summarized in the tables.

## Example I

D,L-methyl-3-chloro-2-methylpropionate (10 mL) was added to a potassium phosphate buffer (27 mL, 0.05 M, pH 7.0). With stirring, at 0° C, lipase of Rhizopus delemar (2 g) was added, after which the pH was maintained at 7.0 by means of automatic titration with 2M of NaOH. After partial conversion (determined via the amount of caustic consumed), the mixture was acidified to a pH of 2.0 and stirred with tert-butylmethylether (40 mL) for 1 hour. After the mixture had been centrifuged (5,000 - 10,000 rpm) the organic layer was removed and the aqueous layer was again extracted with tert-butylmethylether (40 ml).

After centrifugation the two organic layers were combined and washed with a saturated sodium hydrogen carbonate solution (2 x 20 mL). The organic layer was dried with anhydrous MgSO4, filtered, evaporated and distilled at reduced pressure to yield enantiomerically enriched D-(-)-methyl-3-chloro-2-methylpropionate. The combined sodium hydrogen carbonate solutions were acidified to a pH of 2.0 and extracted with tert-butylmethylether (40 mL), after which the organic layer was dried (MgSO4), filtered and evaporated. Distillation at reduced pressure yielded enantiomerically enriched L-(+)-3-chloro-2-methylpropionic acid.

The enantiomeric excess of the two compounds was determined from the measured rotation (via polarimetry) and from this value the conversion was inferred in a known manner (Ching-Shih Chen, loc. cit).

## Example II

The procedure described in example I was repeated, with the understanding that a sodium acetate-acetic acid buffer (0.05 M, pH 5.6) was used instead of the potassium phosphate buffer, the pH being maintained at 5.6.

Example III

The procedure described in example II was repeated, with the understanding that, instead of 27 mL, 24.3 mL of sodium acetate-acetic acid buffer was used, supplemented with 2.7 mL of n-dibutylether as cosolvent.

Example IV

D,L-methyl-3-chloro-2-methylpropionate (156 grams) was added to a potassium phosphate buffer (300 mL, 0.1 M, pH 7.0). With stirring, at 0°C, lipase from Candida cylindraceae (10 grams) was added, after which the pH was maintained at 7.0 as described in example I. After partial conversion, the mixture was acidified and worked up in a manner similar to that described in example I.

Example V

D,L-methyl-3-chloro-2-methylpropionate (136.5 grams) and tert-butylmethylether (100 mL) were added to a potassium phosphate buffer (200 mL, 0.1 M, pH 7.0). With stirring, at 0°C, lipase from Candida cylindraceae (15 grams) was added, after which the pH was maintained at 7.0 as described in example I. After partial conversion the mixture was acidified and worked up in a manner similar to that described in example I.

Comparative examples A-C

D,L-methyl-3-chloro-2-methylpropionate (10 mL) was added to a potassium phosphate buffer (27 mL, 0.05 M, pH 7.0). With stirring, at 28°C, one of the following enzyme preparations was each time added. However, no enzymatic hydrolysis took place (not even after stirring for several weeks).
A: lipase from Penicillium roqueforti (Amano, 4 g);
B: pregastric esterase (Amano, 4 g);
C: protease from fungus Optimase L 660 (Miles Kali, 2 mL) .

Comparative examples D-F

The procedure described in example I was repeated, with the understanding that, instead of lipase obtained from Rhizopus delemar, the indicated amounts of other enzyme preparations were used.

| Comp. example | Source of enzyme prep. (supplier, amount) | conversion (%) | D-(-)-methyl-3-Hal-2-methylpropionate | |
|---|---|---|---|---|
| | | | e.e (%) | e.e 75(%)*) |
| D | lipase from Humicola lanuginosa (Biocatalysts, 2 g) | 16 | 9 | 71 |
| E | acylase from pig's kidney (Sigma, 1 g) | 13 | 5 | 49 |
| F | esterase from pig's liver (Sigma, 2 mL) | 15 | 9 | 74 |

*) e.e 75 (%) indicates the e.e that would theoretically be found in extrapolation of the degree of conversion to 75%.

EP 0 369 553 A1

## Example VI

The procedure described in example I was repeated, with the understanding that, instead of D,L-methyl-3-chloro-2-methylpropionate, an equimolar amount of the corresponding D,L-ethylester, as well as lipase from Rhizopus javanicus were used.

## Example VII

The procedure described in example I was repeated, with the understanding that, instead of D,L-methyl-3-chloro-2-methylpropionate, an equimolar amount of the corresponding 3-bromo-compound was used. The enantiomeric excess of enriched D-(-)-methyl-3-bromo-2-methylpropionate was determined via 400 MHz 1H-NMR with the aid of tris-[3-(heptafluoropropylhydroxymethylene)-d-camphorato]-Europium-III (Eu(hfc)3) in CDCl3.

## Examples VIII through XXV

Microorganisms were cultured in 1-litre Erlenmeyer flasks filled with 500 mL of medium, which, with stirring (150 rpm), were incubated at 28° C. For bacteria the medium consisted of: yeast extract (5 g/L), peptone (5 g/L), starch (10 g/L) and D,L-methyl-3-chloro-2-methylpropionate (1 g/L) with a pH of 7.0. For yeasts and fungi the medium consisted of: yeast extract (3 g/L), peptone (5 g/L), glucose (10 g/L), malt extract (3 g/L) and D,L-methyl-2-chloro-2-methylpropionate with a pH of 5.5. Fifty hours after grafting the cultured cells were harvested by means of centrifugation (10 minutes, 15000 rpm[?*]), washed with potassium phosphate buffer (50 mM, pH 7.0) and resuspended in the same buffer. The cell suspension thus obtained was either used directly for the enzymatic hydrolysis or was first deep-frozen for a short time (-20° C).

The enzymatic hydrolysis was each time carried out as described in example I, using the microorganisms listed in Table 2.

TABLE 1

| Example | Source of the enzyme prep. (supplier, amount) | conversion (%) | d-(-)-methyl-3-Hal-2-methylpropionate e.e (%) *) |
|---------|-----------------------------------------------|----------------|--------------------------------------------------|
| I | Rhizopus delemar (Biocatalysts, 2 g) | 72 | 95 |
| II | Rhizopus delemar (Biocatalysts, 2 g) | 69 | 97 |
| III | Rhizopus delemar (Biocatalysts, 2 g) | 64 | 98 |
| IV | Candida cylindraceae (Sigma, 10 g) | 68 | 95 |
| V | Candida cylindraceae (Sigma, 15 g) | 64 | 98 |
| VI | Rhizopus javanicus (Amano, 2 g) | 73 | 96 |
| VII | Rhizopus delemar (Biocatalysts, 2 g) | 66 | 98 |

*) N.B. Example VI applies to the ethyl ester.

## TABLE 2

| Example | Microorganisms | conversion (%) | D-(-)-methyl-3-chloro-2-methyl methylesterpropionate e.e.(%) |
|---------|----------------|----------------|-------------------------------------------------------------|
| VIII | Rhodococcus erythropolis NCIB 11538 | 59 | 99 |
| IX | Gliocladium viride CBS 22848 | 52 | 96 |
| X | Rhodococcus rhodochrous ATCC 21243 | 59 | 97 |
| XI | Rhodococcus sp. ATCC 4275 | 53 | 95 |
| XII | Mycobacterium phlei ATCC 15610 | 62 | 98 |
| XIII | Nocardia tartaricans ATCC 31190 | 57 | 96 |
| XIV | Rhodococcus rhodochrous ATCC 21197 | 57 | 95 |
| XV | Rhodococcus sp. 1948 | 57 | 95 |
| XVI | Rhodococcus sp. ATCC 19071 | 55 | 99 |
| XVII | Strain TA-2 (NCIB 40069) | 64 | 99 |
| XVIII | Strain TB-2 (NCIB 40070) | 60 | 97 |
| XIX | Erwinia herbicola ATCC 14589 | 56 | 95 |
| XX | Strain EE-1 (NCIB 40074) | 60 | 96 |
| XXI | Pseudomonas fluorescens NV DSM 91 (NCIB 40074) | 58 | 96 |
| XXII | Xanthomonas hyacinthi NV DSM 49 (NCIB 40073) | 66 | 98 |
| XXIII | Bacillus cereus NV DSM 6 (NCIB 40072) | 67 | 96 |
| XXIV | Verticillium glaucum NV DSM 2011 | 70 | 98 |
| XXV | Corynebacterium bovis ATCC 7715 | 64 | 95 |

## Claims

1. Process for the enantioselective preparation of D-(-)-3-Hal-2-methylpropionic acid or derivatives thereof, characterized in that an ester of 3-Hal-2-methylpropionic acid, Hal being a halogen atom, and preferably a chlorine or bromine atom, is subjected to the action of an ester-bond hydrolyzing enzyme preparation so that, at a degree of ester conversion of at most 75%, the remaining ester fraction has an enantiomeric excess of D-ester of at least 95%, and the ester fraction is recovered from the reaction product.

2. Process according to claim 1, characterized in that the hydrolysis is carried out in such a manner that the enantiomeric excess of D-ester is at least 98%.

3. Process according to claim 1 or 2, characterized in that the enantiomeric excess of D-ester is at least 99%.

4. Process according to any one of claims 1-3, characterized in that the enzyme preparation is obtained from a microorganism belonging to the following group: Rhizopus, Mucor, Verticillium, Gliocladium, Rhodococcus, Nocardia, Xanthomonas, Pseudomonas, Bacillus, Corynebacterium, Mycobacterium, Erwinia.

5. Process according to any one of claims 1-4, characterized in that the enzyme preparation is obtained from a microorganism from the following group: Rhizopus delemar, Rhizopus niveus, Rhizopus javanicus, Rhizopus arrhizus, Rhizopus japonicus, Mucor javanicus, Mucor miehei, Verticillium glaucum, Gliocladium viride, Candida cylindriceae, Rhodococcus erythropolis, Rhodococcus rhodochrous, Nocardia tartaricans, Xanthomonas hyacinthi, Pseudomonas fluorescens, Bacillus cereus, Corynebacterium bovis, Mycobacterium phlei, Erwinia herbicola.

6. Process according to any one of claims 1-5, characterised in that the enzyme preparation is obtained from a microorganism from the group consisting of: Verticillium glaucum NV DSM 2011, Gliocladium viride CBS 22848, Rhodococcus erythropolis NCIB 11538, Rhodococcus erythropolis NCIB 11539, Rhodococcus erythropolis NCIB 11540, Rhodococcus rhodochrous ATCC 21243, Rhodococcus rhodochrous ATCC 21197, Rhodococcus sp. ATCC 4275, Rhodococcus sp. ATCC 19071, Rhodococcus sp. ATCC 19148, Nocardia tartaricans ATCC 31190, Xanthomonas hyacinthi NV DSM 49 (NCIB 40073), Pseudomonas fluorescens NV DSM 91 (NCIB 40074), Bacillus cereus NV DSM 6 (NCIB 40072), Corynebacterium bovis ATCC 7715, Mycobacterium phlei ATCC 15610, Strain TA-2 (NCIB 40069), Strain TB-2 (NCIB 40070), Strain EE-1 (NCIB 40071), Erwinia herbicola ATCC 14589.

7. Process according to any one of claims 1-6, characterized in that the ester fraction, which contains an enantiomeric excess of D-ester, is hydrolyzed to D-(-)-3-Hal-2-methylpropionic acid.

8. Use of D-(-)-3-Hal-2-methylpropionic acid or an ester thereof, prepared according to any one of claims 1-7, for the preparation of Captopril or a related compound containing a D-3-mercapto-2-methylpropionic acid radical or a D-3-acylthio-2-methylpropionic acid radical.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 179 603 (ICI)<br>* Claims * | 1 | C 12 P 41/00<br>C 12 P 7/40 |
| A | EP-A-0 206 436 (MASSACHUSETTS INSTITUTE OF TECHNOLOGY)<br>* Claims * | 1 | |
| A | DE-A-3 722 278 (EGIS GYOGYSZERGYAR)<br>* Claims * | 1 | |

TECHNICAL FIELDS SEARCHED (Int. Cl.5)

C 12 P

The present search report has been drawn up for all claims

| Place of search | Date of completion of the search | Examiner |
|---|---|---|
| THE HAGUE | 28-02-1990 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)